(19) Europäisches Patentamt
European Patent Office
Office européen des brevets

(11) Veröffentlichungsnummer: **0 195 917**
**B1**

(12) **EUROPÄISCHE PATENTSCHRIFT**

(45) Veröffentlichungstag der Patentschrift: **18.04.90**

(51) Int. Cl.$^5$: **C 07 D 229/00,** C 08 G 18/79

(21) Anmeldenummer: **86101887.7**

(22) Anmeldetag: **14.02.86**

(54) **Verfahren zur Herstellung von Additionsverbindungen aus 1 Mol dimerisiertem oder niedrigoligodimerisiertem 4,4'-Diisocyanatodiphenylmethan und 2 bis 3 Mol 4,4'-Diisocyanatodiphenylmethan, entsprechende Additionsverbindungen und ihre Verwendung zur Polyurethanherstellung.**

(30) Priorität: **27.02.85 DE 3506834**

(43) Veröffentlichungstag der Anmeldung:
**01.10.86 Patentblatt 86/40**

(45) Bekanntmachung des Hinweises auf die Patenterteilung:
**18.04.90 Patentblatt 90/16**

(84) Benannte Vertragsstaaten:
**BE DE FR GB IT NL**

(56) Entgegenhaltungen:
**EP-A-0 071 898**
**EP-A-0 071 899**
**DE-A-1 445 721**
**DE-A-2 419 968**

(73) Patentinhaber: **BAYER AG**
**D-5090 Leverkusen 1 Bayerwerk (DE)**

(72) Erfinder: **Grögler, Gerhard, Dr.**
**von-Diergardt-Strasse 48**
**D-5090 Leverkusen 1 (DE)**
Erfinder: **Kopp, Richard, Dr.**
**Bilharzstrasse 15**
**D-5000 Koeln 80 (DE)**
Erfinder: **Hess, Heinrich, Dr.**
**Körnerstrasse 5**
**D-5090 Leverkusen 1 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren zur Dimerisierung von 4,4'-Diisocyanatodiphenylmethan aus emulgierter Form in unpolaren Lösungsmitteln unter Bildung von Additionsverbindungen, sowie die erstmals nach diesem Verfahren zugänglichen Molekülverbindungen aus 1 Mol niedermolekularem 4,4'-Diisocyanato-diphenylmethan-uretdion oder seinen niederen Oligomeren mit der idealisierten Formel (I)

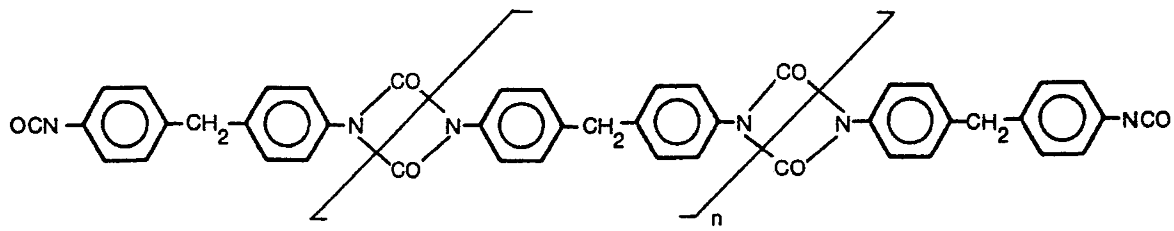

(I)

n 0 bis 0,5 und n = mittlerer Grad der Oligomerisierung des Dimeren; "Oligodimerisierungsgrad", und 2 bis 3 Mol 4,4'-Diisocyanatodiphenylmethan (MDI) (II)

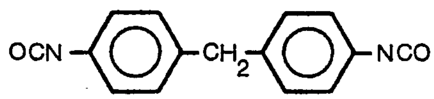

(II)

In dem erfindungsgemäßen Verfahren emulgiert man 4,4'-Diisocyanatodiphenylmethan (II) in geschmolzener Form in bestimmten unpolaren Lösungsmitteln, startet die Dimerisierung in Gegenwart vonn üblichen Dimerisierungskatalysatoren bei Temperaturen oberhalb des Schmelzpunktes von MDI, vorzugsweise bis zu einer oberen Temperaturgrenze von 50 bis 60°C, senkt dann die Temperatur auf <40°C, unterbricht gegebenenfalls die Dimerisierung nach Erreichen eines NCO-Gehaltes von 22,4 bis 26,9% und arbeitet bei niedrigen Temperaturen, vorzugsweise 0 bis 25°C auf.

Die so erhaltene Additionsverbindung, die möglicherweise eine Molekülverbindung aus dimerem 4,4'-Diisocyanatodiphenylmethan (mit gegebenenfalls nur geringen Anteilen an oligomeren Uretdiondiisocyanaten (I) n ≤ 0,5) und 2 bis 3 Mol 4,4'-Diisocyanatodiphenylmethan darstellt, wird als neuer Stoff beansprucht. Weiterhin wird die Verwendung der Additionsverbindung zum gegebenenfalls stufenweisen Aufbau von Polyrethanen beansprucht.

Aromatische Uretdion-diisocyanate ("dimere" Diisocyanate) sowie deren Herstellungsverfahren sind bekannt. Vergleiche Kunststoff-Handbuch, Band VII, Polyurethane, herausgegeben von Vieweg-Höchtlen, Carl-Hanser-Verlag München 1966. Als Dimerisierungskatalysatoren wurden neben Trialkylphosphinen (J. Org. Chem. *8*, 23 (1943)), aromatisch-aliphatische tertiäre Phosphine, Alkyl-diarylphosphine (DE—A 2 452 390), 3- oder 4-substituierte Pyridine (GB—A—821 158), Trialkylphosphite (DE—A 2 349 726) sowie Phosphorigsäure-tris-dialkylamide, (US—A—3 290 288) genannt. Die Dimerisierungsreaktion zum Uretdion kann dabei in Einzelfällen sogar in Substanz erfolgen. Vorwiegend werden aber Lösungsmittel verwendet, die sich gegenüber NCO-Gruppen inert verhalten. Als Lösungsmittel werden z.B. genannt: Benzol, Toluol, Xylol, Chlorbenzol, Nitrobenzol, Aceton, Methylethylketon, Essigester, Dioxan, Tetrahydrofuran, aliphatische Kohlenwasserstoffe, Dimethylformamid und Methylenchlorid. Eine Differenzierung der genannten Lösungsmittel hinsichtlich ihrer Polarität bzw. ihrem Lösungsvermögen gegenüber den Anfangs- bzw. Endprodukten der Reaktion wird in der Patentliteratur nicht vorgenommen.

Daß bei der Dimerisierung von aromatischen Isocyanaten, insbesondere bei reaktiven Dimerisierungskatalysatoren, unerwünschte Nebenprodukte, wie z.B. Isocyanurat, entstehen können, wird bereits in der US—A—2 681 144 beschrieben. Es wird daher empfohlen, die Dimerisierung dann zu unterbrechen, wenn eine gewünschte Dimerisierungsstufe erreicht ist. Als Inhibitoren (Stopper) für den Dimerisierungskatalysator werden z.B. Alkylierungsmittel, insbesondere Cyclohexansulfonsäuremethylester oder Benzylchlorid verwendet.

Aromatische Diisocyanate, bei denen der aromatische Kern in einer oder beiden ortho-Stellungen zur NCO-Gruppe durch Reste R, z.B. Alkylgruppen, substituiert ist, ergeben nach der vorliegenden Literatur Uretdiondiisocyanate mit dem doppelten Molgewicht der Ausgangsverbindungen, z.B. das dimere Toluylendiisocyanat

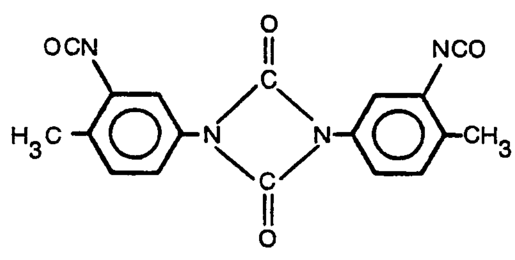

Diese Lehre wird in der Patentliteratur weiterhin übertragen auf solche aromatische Diisocyanate, bei denen die aromatisch gebundene NCO-Gruppe in ortho-Stellung lediglich H-Atome aufweist. Die vielfach in der Patentliteratur übliche Bezeichnung für solche Uretdiondiisocyanate, z.B. auf Basis von 4,4'-Diphenylmethan-diisocyanat lautet daher "Diphenylmethandiisocyanat-Dimer" oder 4,4'-Diisocyanato-diphenylmethan-uretdion, (MDI-Uretdion) oder dimeres MDJ und wird folgerichtig durch die Formel

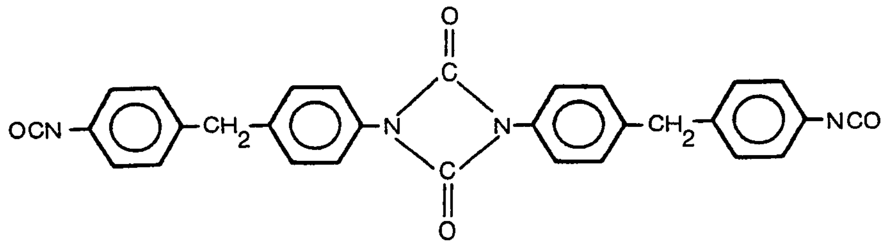

wiedergegeben.

Erstmalig wurden in der DE—A 1 445 721 (US—PS 3 290 288) differenzierte Angaben über oligomere Uretdiondiisocyanatderivate auf Basis von 4,4'-Diphenylmethandiisocyanat gebracht, wobei erwähnt wird, daß bei der Dimerisierung von Diphenylmethandiisocyanat als Endprodukte neben di-, auch tri-, tetra- und pentamere Uretdione erhalten werden

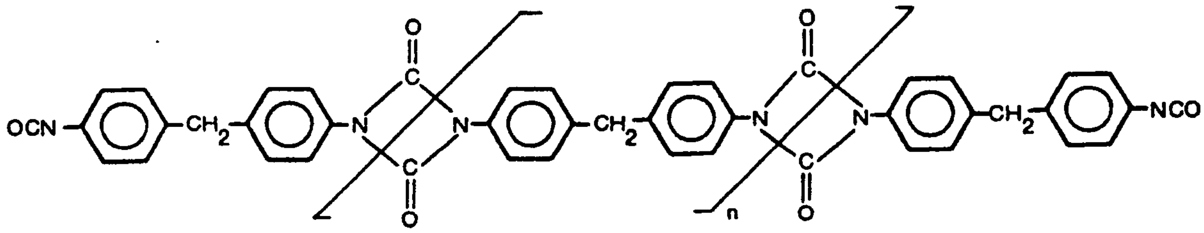

n = 0 bis 5. "Oligomere" Dimere bzw. Uretdione des MDJ enthalten die Uretdiongruppe mehr als einmal im Molekül. Nieder-oligomere Produkte enthalten diese vorzugsweise im Schnitt bei $n \leq 1$ (d.h maximal mit zwei Uredionaruppen).

Die Verwendung von dimerisiertem Diphenylmethandiisocyanat nach obiger Literatur wird in der DE—A 2 419 968 zum Aufbau von hochmolekularen Polyurethanen mit Uretdiongruppen durch Reaktion in hochpolaren Lösungsmitteln wie DMF beschrieben.

Mit zunehmendem Oligodimerisierungsgrad (beispielsweise bei $n > 1$; insbesondere 2,5) nimmt aber infolge erhöhter Schwerlöslichkeit dieser Uretdiondiisocyanate die Reaktivität gegenüber H-aciden Verbindungen deutlich ab, so daß solche höhermolekularen Oligomere für viele Polyurethanreaktionen, besonders wenn sie lösungsmittelfrei bzw. bei Temperaturen unterhalb der (relativ hohen) Schmelzpunkte der Uretdiondiisocyanate durchgeführt werden, praktisch nicht mehr geeignet sind.

In der Patentliteratur wird nur in wenigen Fällen auf die Bedeutung des Lösungsmittels bei der Dimerisierungsreaktion eingegangen.

Einige der in der Patentliteratur zur Dimerisierung von aromatischen Isocyanaten als geeignet angegebenen inerten Lösungsmittel erweisen sich bei der Dimerisierung von Diphenylmethandiisocyanat als unbrauchbar, da hierbei hochmolekulare MDI-Dimer-Gemische ($n > 2,5$) entstehen, die für weitere Polyurethanreaktionen zumeist nicht mehr geeignet sind.

In sehr hochpolaren Lösungsmitteln, wie Dimethylformamid oder Dimethylacetamid entstehen praktisch ausschließlich Poly-Dimere ($n \geq 2,5$) di sich in ihrer Reaktivität und Umsetzbarkeit deutlich von den erfindungsgemäß zugänglichen Verbindungen unterscheiden.

Die Bedeutung des Lösungsmittels für die Dimerisierung von 4,4'-Diisocyanatodiphenylmethan wird erstmalig in der EP—A—0 071 899 offenbart. Es wird dargelegt, daß nicht nur die Art oder die Menge des Katalysators innerhalb der üblichen Grenzen, oder das kontrollierte Abstoppen des Katalysators bei einem bestimmten Dimerisierungsgrad für die selektive Bildung von möglichst niedermolekularen Diphenylmethanuretdion-diisocyanaten verantwortlich ist, sondern es konnte vielmehr nachgewiesen werden, daß insbesondere auch die Wahl des Lösungsmittels von ausschlaggebender Bedeutung ist. So werden bei der Dimerisierung von Diphenylmethan-4,4'-diisocyanat in einem Gemisch von unpolaren und polaren Lösungsmitteln Diphenylmethan-uretdion-diisocyanate mit sehr geringem Oligodimerisierungsgrad n erhalten ($n \leq 1$, vorzugsweise $\leq 0,5$), insbesondere dann, wenn das unpolare Lösungsmittel nur ein geringes Lösungsvermögen für Diphenylmethan-4-4'-diisocyanat (5 bis 25 Gew.-%, vorzugsweise 5 bis 15 Gew.-%) aufweist.

Weiterhin wird in der EP—A—0 071 899 erwähnt, daß man die Dimerisierung von 4,4'-Diisocyanato-diphenylmethan in Lösung in einem unpolaren Lösungsmittel auch in Abmischung mit einem polaren Lösungsmittel durchführen kann. Infolge der großen Verdünnung (hoher Anteil an unpolarem Lösungsmittel) ist dieses Verfahren jedoch wenig wirtschaftlich und darüber hinaus enthalten die entstehenden niedermolekularen Diphenylmethan-uretdion-diisocyanate (je nach Lösungsmittelmenge)

noch eingeschlossene monomere Diphenylmethandiisocyanat-Startkomponente in einem Anteil bis 20 Gew.-%, zumeist von 2 bis 10 Gew.-% zusätzliches MDI bezogen auf Dimeres (I).

Es wurde nun überraschenderweise gefunden, daß man bei der Dimerisierung von 4,4'-Diisocyanatodiphenylmethan (MDI) zu einer Additionsverbindung aus 1 Mol niedermolekularem Uretdiondiisocyanat (mit einem Oligodimerisierungsgrad n = 0 bis 0,5 im MDI-Uretdion (I)) und 2 bis 3 Mol monomerem MDI gelangt, wenn die Dimerisierung von MDI-Monomer nicht aus Lösung, sondern vielmehr aus einer Emulsion von fein verteilten MDI-Tröpfchen in bestimmten unpolaren Lösungsmitteln bei Temperaturen oberhalb des Schmelzpunktes des MDI-Monomeren (II) gestartet wird. Es ist ein entscheidender Vorteil bei dieser Arbeitsweise, daß hierzu nur ein geringer Anteil an Lösungsmitteln zur Herstellung der Emulsion erforderlich ist und daß sich die Additionsverbindungen (enthaltend 40 bis 55,5 Gew.-% MDI-Uretdion (I)/60 bis 44,5% MDI-Monomer (II) bevorzugt 40 bis 50 Gew.-% MDI-Uretdion/60 bis 50 Gew.-% MDI-Monomer) als Feststoff in hoher Ausbeute während der Dimerisierungsreaktion abscheiden. Eine Weiterdimerisierung des MDI-Monomeren erfolgt unter diesen Arbeitsbedingungen nicht oder nur sehr langsam und kann gegebenenfalls durch Abstoppen des Dimerisierungskatalysators ausgeschlossen werden. Die so erhaltene Additionsverbindung kann als festes Ausgangsisocyanat zum Aufbau von Polyurethanen, insbesondere für den stufenweisen Aufbau von Polyurethanen (wegen der verschieden reaktiven NCO-Gruppen in der Additionsverbindung) mit Vorteil eingesetzt werden. Es sind hierdurch Einkomponentenpolyurethansysteme zugänglich, die durch Ausheizen in die hochmolekularen Polyurethane aus einer lagerstabilen Zwischenstufe übergehen.

Gegenstand der Erfindung ist somit ein Verfahren zur Herstellung einer neuartigen Additionsverbindung der idealisierten Formel (III):

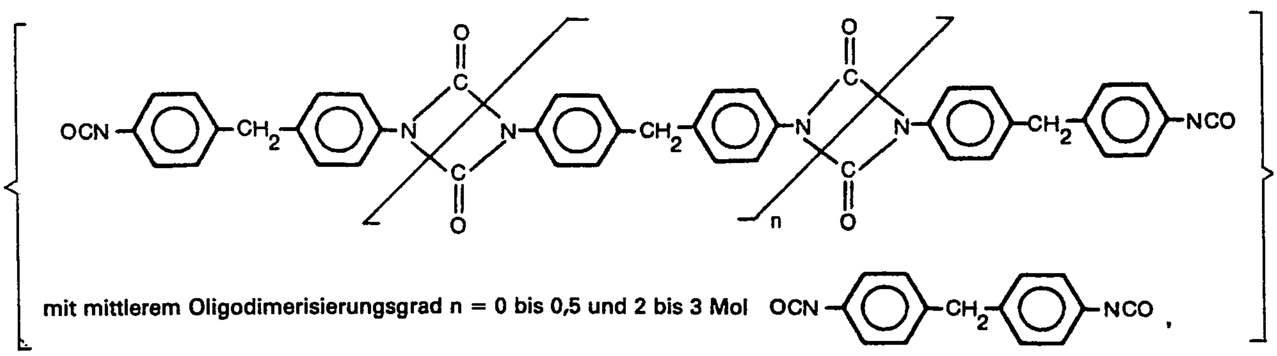

mit mittlerem Oligodimerisierungsgrad n = 0 bis 0,5 und 2 bis 3 Mol

III

aus 1 Mol eines niedermolekularen 4,4'-Diphenylmethanuretdionisocyanats (I), (Oligodimerisierungsgrad n = 0 bis 0,5) und 2 bis 3 Mol 4,4'-Diisocyanatodiphenylmethan (II), durch Dimerisierung von 4,4'-Diisocyanatodiphenylmethan in NCO-inerten Lösungsmitteln in Gegenwart von Dimerisierungskatalysatoren und gegebenenfalls Abstoppern, dadurch gekennzeichnet, daß man die Dimerisierung in einer Emulsion von fein verteiltem, flüssigem Diphenylmethan-4,4'-diisocyanat in einem impolaren Lösungsmittel, das ein möglichst geringes Lösungsvermögen für die Startkomponente Diphenylmethan-4,4'-diisocyanat aufweist, als Emulgiermittel durchführt, wobei die Löslichkeit von Diphenylmethandiisocyanat bei Raumtemperatur in diesem Lösungsmittel höchstens 25 Gew.-%, vorzugsweise 2 bis 15 Gew.-%, besonders bevorzugt 2 bis 12 Gew.-% beträgt und daß die Dimerisierungsreaktion bei 40 bis 60°C insbesondere 40 bis 50°C, gestartet wird (vorzugsweise 5—100 Minuten; insbesondere 5—30 Minuten) und der zunächst leicht exotherm reagierende Ansatz dann der weiteren Dimerisierungsreaktion bei einer Temperatur von 0 bis 40°, bevorzugt 15 bis 30° gehalten oder bevorzugt auf Raumtemperatur abgekühlt wird. Vorzugsweise arbeitet man bei der Isolierung der erfindungsgemäßen Additionsverbindung (III) bei Temperaturen unterhalb von 50°C, vorzugsweise bei Raumtemperatur.

Als Emulgiermittel werden bevorzugt aliphatische und/oder cycloaliphatische Kohlenwasserstoffe, Kohlenwasserstoff-Destillate mit überwiegendem Gehalt an aliphatischen und/oder cycloaliphatische Kohlenwasserstoffen, die besonders bevorzugt eine Dielektrozitätskonstante von unter 2,10 aufweisen, oder Ether eingesetzt.

Die nach dem erfindungsgemäßen Verfahren hergestellte, feste Polyisocyanatadditionsverbindung (III) stellt eine interessante Ausgangskomponente für Polyurethane dar, da sie zwei verschiedenartig reagierende Diisocyanate enthält.

Gegenstand der vorliegenden Erfindung ist daher auch die Additionsverbindung (III) aus 1 Mol eines niedermolekularen MDI-Uretdiondiisocyanats (I; n = 0 bis 0,5) und 2 bis 3 Mol 4,4'-Diisocyanatodiphenylmethan (II), wobei der Gehalt dieser Additionsverbindung an freiem NCO im Bereich von 22,4 bis 26,9%, vorzugsweise 24,4 bis 26% liegt und nach dem beanspruchten Verfahren erhältlich ist.

Ein weiterer Gegenstand der Erfindung ist die Verwendung dieser Additionsverbindung (III) als Polyisocyanatkomponente zum Aufbau von Polyurethanen, insbesondere lagerstabilen Einkomponentenpolyurethanreaktivmischungen.

Zur Herstellung der Additionsverbindung (III) werden als Dimerisierungskatalysatoren die üblichen

Katalysatoren, wie sie bereits aufgeführt worden sind, eingesetzt, insbesondere jedoch Trialkylphosphine, wie Triethylphosphin oder Tributylphosphin. Die Katalysatoren werden in üblichen Mengen, z.B. 0,05 bis 5 Gew.-%, vorzugsweise jedoch 0,1 bis 1,0 Gew.-% und besonders bevorzugt bei 0,1 bis 0,35 Gew.-% eingesetzt.

Als "unpolare" Emulgiermedien kommen diejenigen Verbindungen in Betracht, die ein möglichst geringes Lösungsvermögen für die Startkomponente MDI aufweisen. Dies sind aliphatische und/oder cycloaliphatische Kohlenwasserstoffe wie Hexan, Octan, Cyclopentan, Cyclododecan, sowie entsprechende Kohlenwasserstoff-Destillate mit überwiegendem Gehalt an aliphatischen und/oder cycloaliphatischen Kohlenwasserstoffen, z.B. Petrolether, Waschbenzin oder Ligroin. Die Dielektrizitätskonstante derartiger Lösungsmittel liegt im allgemeinen unter 2,10.

Weiterhin geeignet sind auch Emulgiermedien ("Lösungsmittel"), die eine Ethergruppe im Molekül besitzen, wie z.B. Di-n-propylether, Diisopropylether, Methyl-tert.-butylether, Methyl-tert.-amylether, besonders aber Diisopropylether.

Zur Ausführung des erfindungsgemäßen Verfahrens wird die Startkomponente MDI (II) in geschmolzener Form dem Emulgiermittel ("Lösungsmittel") zugesetzt, wobei die Temperatur des Emulgiermittels oberhalb des Schmelzpunktes von MDI liegt.

Zur einwandfreien Herstellung der MDI-Emulsion ist im allgemeinen ein kräftiges Durchmischen der vorliegenden zwei flüssigen Phasen erforderlich (Rührwerk). Gegebenenfalls werden hierbei die in der Fachliteratur bereits beschriebenen Emulgierhilfsmittel mitverwendet. Man kann aber auch so verfahren, daß man eine Mischung von pulverisiertem MDI und flüssigem Emulgiermittel unter heftigem Rühren auf Temperaturen oberhalb des Schmelzpunktes von MDI bringt und dann der Emulsion von (II) im Emulgiermittel den Dimerisierungskatalysator zusetzt.

Die Dimerisierung wird zunächst bei Temperaturen oberhalb des Schmelzpunktes von MDI, z.B. bei 40 bis 60°C, insbesondere 40 bis 50°C, gestartet und der Ansatz anschließend auf einer Reaktionstemperatur von 0 bis maximal 40°C, vorzugsweise 15 bis 30°C gehalten, insbesondere auf Raumtemperatur abgekühlt. Die Dimerisierungsreaktion nimmt unter diesen Bedingungen einen Zeitraum von 1 bis 6 Stunden in Anspruch.

Bei einer Temperatur von $>40°C$ während der gesamten Dimerisierungsreaktion wird zwar bei kürzerer Reaktionszeit mehr festes Addukt abgeschieden, man erhält jedoch dann je nach Wahl des Lösungsmittels (Emulgiermittels) zuweilen Additionsprodukte mit geringeren NCO-Gehalten (d.h. eine zunehmende Oligodimerisierung mit Werten von $n > 0,5$) und NCO-Gehalten unter der erfindungsgemäß beanspruchten Grenze.

Die Menge des Emulgiermittels kann in breiten Grenzen variieren, es ist aber vorteilhaft, dann geringere Mengen des Emulgiermittels zu verwenden, wenn sie ein relativ gutes Lösungsvermögen (z.B.) 10 Gew.-% MDI in der Lösung bei Raumtemperatur) für MDI aufweisen. Selbstverständlich muß die Menge des Emulgiermittels auch so gewählt sein, daß die aus der Emulsion durch Dimerisierung entstandene Feststoff-Suspension der Additionsverbindung noch rührbar bleibt, was auch die Isolierung des Additionsprodukts z.B. durch Filtration erleichtert. Im allgemeinen werden auf 1 Gew.-Teil MDI 1 bis 5 Gew.-Teile, vorzugsweise 2 bis 3 Gew.-Teile Emulgiermittel verwendet.

Die weiteren Dimerisierungsbedingungen, wie Wahl und Menge des Katalysators, Temperaturführung, Reaktionszeiten, Abstoppen der Katalysatoren wurden in der vorliegenden Literatur hinreichend beschrieben.

Das Ausfallen der festen Additionsverbindung (III) aus 1 Mol niedermolekularen MDI-Dimer und 2 bis 3 Mol MDI beginnt bei den genannten Reaktionstemperaturen kurz nach Zugabe des Katalysators.

Aufeinanderfolgende Probeentnahmen zeigen, daß sich auch bei hohem Überschuß von MDI bei Beginn der Dimerisierungsreaktion stets eine Additionsverbindung aus der Emulsion abscheidet. Eine weitere Dimerisierung des im Molekülverband befindlichen MDI-Monomeren erfolgt unter diesen Arbeitsbedingungen (niedrige Temperatur, z.B. Raumtemperatur) nicht mehr. Trotzdem ist es empfehlenswert, die Reaktion nach Beendigung der Dimerisierung abzustoppen, insbesondere dann, wenn das Emulgiermittel ein "relativ gutes" Lösungsvermögen für MDI-Monomere (z.B. $>10\%$ MDI gelöst) aufweist.

Für eine definierte Molekülverbindung aus 1 Mol niedermolekularem MDI-Dimer (I; $n = 0$) und 2 Mol MDI (50 Gew.-% MDI-Dimer, 50 Gew.-% MDI) berechnet sich ein NCO-Gehalt von 25,2 Gew.-% freies NCO; für eine definierte Molekülverbindung aus (I) und 3 Mol MDI (II) berechnet sich ein Gehalt von 26,9% freies NCO. Die in der Praxis gefundenen Werte können jedoch zuweilen etwas höher liegen, da insbesondere je nach Wahl des Lösungsmittels das niedermolekulare MDI-Dimer (für MDI-Dimer (I) mit $n = 0$ wird ein NCO-Wert von 16,8% freies NCO berechnet) noch zusätzliches, monomeres MDI (II) mit einschließen kann. Dieser Anteil kann bis zu etwa 20 Gew.-% zusätzliches freies MDI betragen, bezogen auf MDI-Dimeres, vorzugsweise bis 10 Gew.-%, was den NCO-Gehalt des Dimeren bis auf 19,6 bzw. 18,3 erhöhen kann.

Diese Form des 10 bis 20 gew.-%igen Einschlusses von monomerem MDI in dimerisiertem MDI bildet sich insbesondere auch bei Dimerisierungsreaktionen nach dem Stand der Technik, d.h. Dimerisierung aus Lösung von MDI und ist auch durch 30-minütige Behandlung mit Toluol bei 50 bis 60° nur schwer extrahierbar. Demzufolge werden auch im erfindungsgemäßen Verfahren der Dimerisierungsreaktion aus Emulsion zuweilen Additionsprodukte aus MDI-Dimer (I) und MDI erhalten, die etwas erhöhte NCO-Gehalte aufweisen (bis 27 Gew.-%; vgl. Beispiele).

Additionsverbindungen mit einem NCO-Gehalt von kleiner als 22,4% NCO (entsprechend einem Dimeren (I) mit <13,44% freiem NCO) sind für die erfindungsgemäße Verwendung als Polyurethankomponente wenig geeignet. Sie enthalten dann bereits sehr viel höher oligomeres MDI-Dimeres (n > 0,5), das infolge seiner Schwerlöslichkeit nur sehr schwer mit H-aktiven Verbindungen zur Reaktion gebracht werden kann. Solche Additionsverbindungen mit zu niedrigem NCO-Gehalt entstehen insbesondere, wenn polare Lösungsmittel als Emulgiermittel verwendet oder die Reaktionstemperatur zur hoch gewählt wurde oder die Reaktionszeit in polaren Lösungsmitteln ohne Abstoppung der Dimerisierungsreaktion zu lange war.

Es soll daher auch in der Aufarbeitungsstufe der Additionsverbindung aus 1 Mol niedermolekularem MDI-Dimer und 2 bis 3 Mol MDI-Monomeren eine nur schonende Temperaturbehandlung erfolgen, da sonst ebenfalls eine weitere Uretdionbildungsreaktion aus den freien NCO-Gruppen des dimeren MDI sowie den NCO-Gruppen des monomeren MDI erfolgt und sich dann polymere Uretdione (I; n > 0,5) bilden. Letztere sind insbesondere bei n ≥ 1, außerordentlich schwer löslich und enthalten wenig freie NCO-Gruppen und lassen sich außerordentlich schwer weiter umsetzen. Dies dürfte auch die bischer ausstehende Nutzung sogenannter "Dimerer" des Diphenylmethan-4,4'-diisocyanats erklären, da diese "Dimeren" nach bisherigen Methoden in nur polymerer oder hocholigomerisierter (und nicht in dimerer oder wenig oligomerisierter Form (n ≤ 0,5)) erhalten wurden.

Die erfindungsgemäße Molekülverbindung aus 1 Mol niedermolekularem MDI-Dimer ((I); n ≤ 0,5) und 2 bis 3 Mol MDI-Monomer läßt sich mit Vorteil zum Aufbau von Polyurethansystemen verwenden. Sie eignet sich insbesondere auch zur Herstellung von Einkomponentenreaktivsystemen gemäß DE—A 3 230 757 und DE—A 34 19 429.

Als übliche Polyurethan-Aufbaukomponenten können die dort aufgeführten höhermolekularen Verbindungen mit Zerewitinoff-aktiven H-Atomen, insbesondere Polyole und/oder Polyamine, gegebenenfalls weitere Polyisocyanate, niedermolekularen Kettenverlängerungsmittel oder Vernetzer, insbesondere Wasser, Diole und/oder aromatische Diamine, sowie die üblichen Zusatzstoffe, Hilfsmittel und Katalysatoren verwendet werden. Als Polyurethen aufbauende Verfahren sind die üblichen, wohlbekannten Ein- oder Mehrstufenverfahren geeignet — insbesondere lassen sich die Additionsverbindungen (III) auch in einer Art Einkomponentenreaktivsystem verwenden, da der monomere MDI-Anteil (II) vorweg zu bereits höhermolekularen Polyurethanen führt, die aber erst (gegebenenfalls aus dieser lagerstabilen Stufe heraus) unter Miteinbeziehung der Dimeren (I) zu den hochmolekularen PU-Endprodukten abreagieren.

Geeignete Ausgangsmaterialien wie höhermolekulare, H-aktive Verbindungen, Kettenverlängerungsmittel oder Vernetzer, gegebenenfalls weitere Diisocyanate sowie Zusatzmittel werden ausführlich in den DE—OS 2 854 384 und DE—A 2 920 501 sowie DE—A 3 230 757 und EP—A 71139 aufgezählt.

## Beispiele

### Beispiel 1

Bestimmung der Löslichkeit von 4,4'-Diisocyanatodiphenylmethan (MDI) (Vorversuche)

Die Löslichkeit von MDI in einigen unpolaren Lösungsmitteln wurde bei Raumtemperatur (25°C) und bei 40°C über NCO-Titration bestimmt. Dabei wurden 100 g Lösungsmittel mit 50 g MDI bei der betreffenden Temperatur ½ h unter Rühren vermischt, die Mischung filtriert bzw. dekantiert und der NCO-Gehalt der klaren Lösung bestimmt. Aus dem NCO-Gehalt ergibt sich die MDI-Konzentration im unpolaren Lösungsmittel.

| | bei Raumtemperatur | | bei 40°C | |
|---|---|---|---|---|
| Lösungsmittel | NCO-Geh. % | MDI g/100 g LM | NCO-Geh. % | MDI g/100 g LM |
| Petrolether | 3,75 | 11,2 | 9,6 | 28,6 |
| Waschbenzin | 3,95 | 11,8 | 7,0 | 20,8 |
| Hexan | 4,0 | 11,9 | 11,8 | 35,1 |
| Ligroin | 6,1 | 18,2 | 11,1 | 33,0 |
| Cyclohexan | 7,05 | 21,0 | 9,95 | 29,6 |
| Di-isopropylether | 10,2 | 30,4 | 11,4 | 32,9 |

Beispiel 2

a) Dimerisierung von MDI in unpolaren und polaren Lösungsmitteln (erfindungsgemäß und Vergleich)

100 g der angegebenen Lösungsmittel werden auf 40°C erwärmt und mit 50 g MDI in geschmolzener Form (Fp.: 40 bis 45°C) unter Rühren vermischt. Danach erfolgt Zugabe von 0,3 g Tributylphosphin. Die Heizung wird nunmehr entfernt. Bereits nach kurzer Zeit beobachtet man eine kristalline Abscheidung des festen, dimerhaltigen MDI-Additionsproduktes. Der Reaktionsansatz wird noch 4 h weitergerührt, wobei sich die Temperatur langsam auf 30 bis 20°C erniedrigt. Der feste Niederschlag wird abgesaugt, mit wenig Lösungsmittel gewaschen und im Vakuum bei 40 bis 50°C getrocknet. Danach wird der NCO-Gehalt dieser Probe bestimmt.

| Nr. | Lösungsmittel | | Ausbeute % d.Th. | Titration NCO-Gehalt % |
|---|---|---|---|---|
| 1 | Petrolether | | 95 | 26,5 |
| 2 | Waschbenzin | wenig polare | 90 | 25,9 |
| 3 | Hexan | Lösungsmittel; | 86 | 25,8 |
| 4 | Ligroin | entsprechend der | 94 | 26,0 |
| 5 | Cyclohexan | Erfindung | 94 | 25,7 |
| 6 | Di-isopropylether | | 92 | 24,8 |
| 7 | Chlorbenzol | polare Lösungs- | 92 | 17,8 |
| 8 | Essigsäureethylester | mittel (*nicht* erfindungsgemäß als | 90 | 14,5 z.T. unlösl. |
| 9 | Methylglykoletheracetat | Vergleich) | 94 | 18,1 |
| 10 | Benzonitril | | 96 | 13,5 z.T. unlösl. |
| 11 | Aceton | | 95 | 14,5 z.T. unlösl. |

Während der Dimerisierung von MDI in Abmischung mit den erfindungsgemäß zu verwendenden unpolaren Lösungsmitteln zu einem Additionsprodukt (III) (NCO-Gehalt: 24,8 bis 26,5; vgl. 1 bis 6) führt, erhält man unter den gleichen Bedingungen in polaren Lösungsmitteln zum Teil höhermolekulare dimere MDI-Oligomere mit Werten von $n > 0,5$ bzw. $> 1,0$) (vgl. 7 bis 10). Im Gegensatz zu den unpolaren Lösungsmitteln ist MDI in den genannten polaren Lösungsmitteln sehr gut löslich und führt daher zu gegebenenfalls höheroligomeren Dimerisierungsprodukten, jedoch nicht zu den erfindungsgemäßen Additionsverbindungen (III). In ähnlicher Weise entstehen auch aus (hoch-)verdünnten Lösungen (anstelle von (höher-)konzentrierten Emulsionen) von MDI in unpolaren Lösungsmitteln höheroligomere Dimerisierungsprodukte und nicht die Additionsverbindungen (III).

Zeitlicher Verlauf der Dimerisierung von MDI aus Emulsionen in unpolaren Lösungsmitteln

Zu 500 ml der genannten Lösungsmittel wurden bei 40°C unter starkem Rühren 250 g (1,0 Mol) geschmolzenes MDI zugesetzt. Danach erfolgte Zugabe von 0,5 g Tributylphosphin. Die äußere Heizung wurde entfernt. Infolge der leicht exotherm ablaufenden Reaktion verblieb die Temperatur noch einige Zeit bei 35 bis 40°C, erniedrigte sich aber allmählich auf Raumtemperatur (20 bis 25°C). In Zeitabständen von einer Stunde wurden 50 ml-Proben der entstehenden Suspension entnommen, der Dimerisierungskatalysator mit einigen Tropfen Toluolsulfonsäureethylester desaktiviert, der Feststoff abgesaugt, gewaschen und bei niederen Temperaturen getrocknet. Danach wurden Gewicht und NCO-Gehalt dieser Feststoffproben bestimmt.

Nach einer Reaktionszeit von 6 h wurde bei dem gesamten Ansatz mittels 0,5 g Toluolsulfonsäureethylester (Tosylester) die Dimerisierung unterbrochen und nach Lagerung über Nacht aufgearbeitet (A).

Bei einer weiteren Versuchsreihe in den genannten Lösungsmitteln wurde der Reaktionsansatz *nicht* mit Tosylester abgestoppt, sonst aber wie in obiger Serie und unter gleichen Bedingungen untersucht (B).

Darüber hinaus wurde durch Extraktion mit Toluol ($\frac{1}{2}$ h/60°C) aus A) das MDI-Dimer isoliert und der NCO-Gehalt über Titration bestimmt (C).

| Proben-entnahme nach h | unpolares Lösungsmittel Hexan g | Festst./NCO % | Cyclohexan g | Festst./NCO % | Petrolether g | Festst./NCO % | Ligroin g | Festst./NCO % | Di-isopropylether g | Festst./NCO % |
|---|---|---|---|---|---|---|---|---|---|---|
| 1 | 4,1 | (27,3) | 5,9 | 26,5 | 4,6 | (27,4) | 5,6 | 26,5 | 2,7 | 27,0 |
| 2 | 7,9 | 26,7 | 6,3 | 25,8 | 5,5 | 27,0 | 6,8 | 26,9 | 4,8 | 26,5 |
| 3 | 8,2 | 26,5 | 8,2 | 25,7 | 6,7 | 26,5 | 7,8 | 25,0 | 5,5 | 26,1 |
| 4 | 8,7 | 26,9 | 8,3 | 25,3 | 8,3 | 26,5 | 8,8 | 26,0 | 6,1 | 26,4 |
| 5 | 9,3 | 25,8 | 8,8 | 26,0 | 8,2 | 26,5 | 9,2 | 25,5 | 6,2 | 25,9 |
| 6 | 10,0 | 25,6 | 11,0 | 25,5 | 8,5 | 26,8 | 9,7 | 25,6 | 6,4 | 25,8 |

| Proben-entnahme nach h | unpolares Lösungsmittel Hexan g | Festst./NCO % | Cyclohexan g | Festst./NCO % | Petrolether g | Festst./NCO % | Ligroin g | Festst./NCO % | Di-isopropylether g | Festst./NCO % |
|---|---|---|---|---|---|---|---|---|---|---|
| *Weg A*: (Dimerisierung *unter*brochen) | | | | | | | | | | |
| (Ausbeute) in g | 200 g | 25,2 | 215 g | 25,2 | 215 g | 26,7 | 212 g | 25,2 | 205 g | 24,8 |
| in % d. Th. | (88%) | | (86%) | | (86%) | | (85%) | | (82%) | |
| MDI-Dimer (C) (extrahiert aus A) | | | | | | | | | | |
| % NCO | | 17,2 | nich gemessen | | | 17,8 | | 16,8 | | 16,9 |
| *Weg B*: (Dimerisierung *nicht* unterbrochen) | | | | | | | | | | |
| Ausbeute in g | 210 g | 24,5 | 205 g | 24,8 | 202 g | 25,8 | 200 g | 25,0 | 190 g | 24,5 |
| in % d.Th. | (84%) | | (82%) | | (81%) | | (80%) | | (76%) | |

Aus diesem Ergebnis ist zu entnehmen, daß sowohl bei sehr hohem Überschuß von MDI bei Beginn der Dimerisierungsreaktion wie auch bei hohem Anteil von MDI-Dimer und relativ geringem MDI-Gehalt bei Beendigung der Dimerisierungsreaktion stets eine Additionsverbindung mit einem NCO-Gehalt von 24,4 bis 27% (NCO $_{ber,}$ für {MM/2 MDI} = 25,2%; {für MM/3 MDI} = 26,9%) gebildet wird. Die Extraktion mit Toluol ($\frac{1}{2}$ h/60°C) und anschließende Isolierung des unlöslichen MM-Anteils ergibt, daß in den genannten Lösungsmitteln MDI-Dimere entstanden sind, die z.T. etwas erhöhten NCO-Gehalt aufweisen (z.B. Hexan, Petrolether). Es ist zu vermuten, daß hierbei eingeschlossenes, nicht extrahierbares aber noch titrierbares MDI in einer Größenordnung von bis <20 Gew.-% vorliegt. Bei den zugrundeliegenden Additionsverbindungen mit den 2 bis 3 Mol MDI erscheint der erhöhte NCO-Gehalt des dimeren MDI (MM) dann auch in der Endbilanz, so daß sie ebenfalls einen etwa um diesen Betrag erhöhten NCO-Wert aufweisen können.

Aus den angeführten Versuchsbeispielen ist auch erkennbar, daß in bestimmten Lösungsmitteln (z.B. Hexan) eine Unterbrechung der Dimerisierung (durch Zugabe von üblichen Abstoppern, z.B. Tosylester) zu definierteren Additionsprodukten führt, weshalb die Abstoppung der Dimerisierung bevorzugt durchgeführt wird.

Beispiel 4

Dimerisierung von MDI bei erhöhter Temperatur

Die Dimerisierung von MDI erfolgte im Gegensatz zu den in Beispiel 3 angegebenen Versuchen nach Start bei 40° nicht im weiteren Verlauf bei Raumtemperatur (20—25°C), sondern über den gesamten Reaktionsverlauf bei 40°C. Als "Lösungsmittel" wurde Petrolether und Ligroin verwendet. Die Aufarbeitung der Reaktionsansätze (Probenentnahme, Isolierung des Additionsproduktes, Extraktion) wurde wie in Beispiel 3 durchgeführt.

| Probe nach h | Petrolether g Festst./50 ml | % NCO | Ligroin g Festst./50 ml | % NCO |
|---|---|---|---|---|
| 2 | 10,0 | (27,2)* | 5,0 | 25,8 |
| 3 | 10,2 | (27,1)* | 6,2 | 25,1 |
| 4 | 11,2 | 26,8 | 9,1 | 24,2 |
| 5 | 10,8 | 26,4 | 8,8 | 22,8 |
| 6 | 11,2 | 26,6 | 8,5 | 22,2** |
| Extraktion des Additionspr. nach 6 h | | 17,8 | | 12,0** |

*) nicht erfindungsgemaß
**) z.T. unlöslich bei Titration (nicht erfindungsgemäß)

Während die Dimerisierung von MDI in Petrolether bei 40°C noch zu einer definierten Additionsverbindung mit einem NCO-Gehalt von 26,6% (nach Extraktion NCO-Gehalt = 17,8% im Dimeren (I)) führt, ist die in Ligroin bei 40°C Dauer-Dimerisierungstemperatur entstandene Verbindung für weitere Polyurethanreaktionen nach dem erfindungsgemäßen Verfahren praktisch ungeeignet. Das im Molekülverband der Additionsverbindung (III) vorliegende MDI-Dimer (I) liegt dann in bereits zu hochmolekularer Form vor (n >> 0,5; freies NCO = 12%) und kann mit den üblichen H-aktiven Verbindungen infolge seiner Schwerlöslichkeit nicht mehr befriedigend und vollständig zur Reaktion gebracht werden. Daher wird die Herstellung der Additionsverbindung (III) nach Start der Dimerisierungsreaktion aus der Emulsion bei 40 bis 60°C dann bevorzugt bei niederen Temperaturen (vorzugsweise Raumtemperatur) in der weiteren Dimerisierungsphase durchgeführt, um eine gegebenenfalls zu weitgehende Oligodimerisierung (z.B. n > 0,5) auszuschließen. Die bevorzugte Dimerisierungstemperatur kann durch entsprechende Vorversuche in dem ausgewählten Lösungsmittel leicht bestimmt werden.

### Beispiel 5

Charakterisierung der erfindungsgemäßen Molekülverbindung

Der NCO-Gehalt der erfindungsgemäßen Additionsverbindungen (III) aus 1 Mol MDI-Dimer (I; n = 0 bis n = 0,5) und 2 bis 3 Mol MDI liegt im Bereich von 22,4 bis 26,9% freies NCO (ber. für (III); n = 0, 2 Mol MDI = 25,2% bei 16,8% NCO-Anteil im Dimer mit n = 0; bzw. bei einem berechneten NCO-Gehalt von 26,9% freies NCO bei (III), n = 0 und 3 Mol MDI).

Bei der Titration mit Di-n-butylamin in Aceton bei Raumtemperatur erfolgt klare Lösung der erfindungsgemäßen Additionsverbindungen (III). Im IR-Spektrum sind die NCO- (2250 $cm^{-1}$) und die Uretdionbande (1780 $cm^{-1}$) deutlich erkennbar.

Wie nachstehender Versuch zeigt, läßt sich das MDI-Monomer (II) mit polaren Lösungsmitteln, insbesondere in der Wärme, leicht aus dem Molekülverband extrahieren. Als Rückstand verbleibt dann ein niedermolekulares MDI-Dimer.

Extraktionsbedingungen:

100 g Toluol
20 g MM/2 MDI (NCO 25,3%)

30 min/60°C.

Rückstand/fest: 9,8 g; NCO = 16,8% (ber. MDI-Dim. 16,8% bei n = 0)
Filtrat eingeengt: 10,2 g: NCO 32,8% (ber. MDI 33,6%)

$$\text{Verhältnis: } \frac{\text{MM (Mol 500)}}{\text{MDI Mol 250}} = \frac{9,8}{10,2} = \frac{19,6\text{ mMol}}{40,8\text{ mMol}} = \frac{1}{2,08}$$

Bei der üblichen Aufheizgeschwindigkeit bei der Schmelzpunkbestimmung der erfindungsgemäßen Additionsverbindung (III) ist im Bereich von 40 bis 45°C (Fp. von MDI) nur in wenigen Fällen ein leichtes Sintern der Verbindung erkennbar. Oberhalb von 250°C beginnt allmähliche Zersetzung des Produkts. Daß kein deutlicher Schmelzbereich bei 40 bis 45°C zu beobachten ist, könnte dadurch erklärt werden, daß das

im Molekülverband mit MDI-Dimer vorliegende MDI bei erhöhter Temperatur kontinuierlich in höhermolekulare Oligomere übergeht. Wird z.B. eine {MM/2 MDI}-Probe (NCO = 25,3%) über längere Zeit bei 120°C getempert, so erfolgt deutlicher NCO-Abfall und man erhält nach einigen Stunden ein unlösliches höheroligomeres MDI-Dimer:

Übergang der Additionsverbindung (III) in Oligodimere (II, n >> 0,5) durch Temperung bei 120°C:

| Zeit/h | % NCO | |
|---|---|---|
| 0 | 25,3 | |
| 1 | 24,8 | |
| 2 | 23,4 | |
| 5 | 18,5 | |
| 10 | 12,1 | bei der Titration |
| 13 | 10,7 | unlösliche Bestandteile |

Wegen der thermischen Umwandlung wird die Aufarbeitung der Molekülverbindung daher bei möglichst niedrigen Temperaturen (z.B. 0—40°C, vorzugsweise 0—25°C) vorgenommen.

Beispiel 6

Herstellung der erfindungsgemäßen Molekülverbindung

Zu 2000 ml, auf 40°C erwärmtem Hexan wurden 1000 g geschmolzenes MDI (40—45°C) unter starkem Rühren eingemischt. Dieser MDI/Hexan-Emulsion wurden 1,5 g Tributylphosphin zugesetzt. Die Heizung wurde danach entfernt. Kurz nach Zugabe des Dimerisierungskatalysators (wenige Minuten) begann die Abscheidung der Molekülverbindung in Form kleiner Kristalle. Es wurde weitere 5 h gerührt, wobei sich die Reaktionstemperatur allmählich auf 30—25°C erniedrigte. Danach wurde mit 1,5 g Tosylester die Dimerisierung abgestoppt. Man ließ den Ansatz mehrere Stunden bei Raumtemperatur stehen, um eine völlige Abscheidung der Verbindung zu gewährleisten. Danach saugte man das feste Material ab, wusch mit wenig Hexan nach und trocknete den Rückstand bei 30—50°C im Vakuum. Man erhielt 940 g (94% d.Th.) der festen Molekülverbindung mit einem NCO-Gehalt von 25,4% (ber. 25,2% für 1:2) und einem Zersetzungspunkt oberhalb von 250°C.

Auch ohne Abstoppen des Dimerisierungskatalysators führt die Dimerisierung zu der definierten Verbindung, da diese sich infolge ihrer Schwerlöslichkeit in Hexan einer weiteren Dimerisierung durch Ausfällung entzieht.

Verwendungsbeispiele:

Beispiel 7

Polyurethan/-2-Stufen-Aufbau/Polyether

Zu einer Mischung von 500 g (0,25 Mol) eines linearen Polypropylenglykolethers (Molgewicht 2000, OH-Zahl: 56), 44,5 g (0,25 Mol) 2,4- und 2,6-Diamino-3,5-diethyltoluol-Gemisch (in Molverhältnis 65/35) (DEDTA) und 0,5 g Pb-octoat (50 %ige Lösung in Benzin) wurden 160 g der Additionsverbindung vom NCO-Gehalt = 26,2% zugesetzt und homogen verrührt. Nach kurzer Zeit erfolgte bei Raumtemperatur Vorreaktion des aromatischen Diamins DEDTA vorwiegend mit dem in der Additionsverbindung vorliegenden MDI (exotherm) und man erhielt nach wenigen Stunden ein bei Raumtemperatur zähplastisches PUR-Voraddukt. In dieser Additonsstufe trat praktisch nur MDI-Monomer (II) in Reaktion, während das infolge seiner Schwerlöslichkeit langsamer reagierende MDI-Dimer (I) noch als inertes Polyisocyanat neben dem Polyether vorlag. Dieses reagierte erst bei höheren Temperaturen und in Gegenwart von Katalysatoren (Pb-octoat). Die Endverfestigung des zäh-plastischen Voraddukts erzielte man daher erst bei einer Reaktionstemperatur von 120—130°C, wobei die Verfestigung des Voraddukts entweder sofort nach der Herstellung oder auch zu einem beliebigen Zeitpunkt unter Druck und Formgebung erfolgen kann. Man erhielt nach Endverfestigung ein elastisches steifes PUR- Material mit einer Härte von 90—95 A.

Beispiel 8

Polyurethan/-2-Stufen-Aufbau/Polyester

Zu einer Schmelze (ca. 50—60°C) von 200 g (0,1 Mol) eines linearen Polyesters aus Adipinsäure und Ethylenglykol mit einem Mol-Gewicht von 2000 (OH-Zahl = 56), wurden 43,3 (0,24 Mol) 2,4- und 2,6-Diamino-3,5- di-ethyltoluol, (Isomerengemisch 65/35) 0,1 g Pb-octoat (Lösung) und 0,1 g FORMREZ UL 29 (S-haltiger Sn-Katalysator, Fa. Witco/USA) und danach 114,9 g des Additionsproduktes (III, 26% NCO) zugerührt und rasch vermischt. Nach kurzer Zeit erfolgte die Vorreaktion des aromatischen Diamins mit dem in der Additionsverbindung vorliegenden MDI in der Polyesterschmelze. Nach dem Abkühlen der Schmelze auf Raumtemperatur erhielt man ein festes, aber sprödes und brechbares Voraddukt, das bei Raumtemperatur unbegrenzt lagerstabil ist. In dieser Additionsstufe kam das aromatische Diamin (0,24 Mol) nur mit dem vorliegenden MDI (0,24 Mol) ohne Mitwirkung des MDI-Dimers zur Reaktion.

Mit geeigneten Mahlapparaturen kann das feste Voraddukt in Granulatform überführt werden. Dieses kann sofort oder auch zu einem beliebigen Zeitpunkt bei einer Reaktionstemperatur von 120—140°C unter

Druck und Formgebung zu einem elastischen, steifen PUR-Material mit einer Härte von 95 A verfestigt werden. In dieser Verfestigungsphase bei erhöhter Temperatur und unter Mitwirkung von Katalysatoren erfolgte die Polyaddition von MDI-Dimer mit dem höherschmelzenden Polyester (Fp. ca. 50°C). Man erhielt ein Elastomeres mit guten Eigenschaften.

**Patentansprüche**

1. Verfahren zur Herstellung einer neuartigen Additionsverbindung der idealisierten Formel (III)

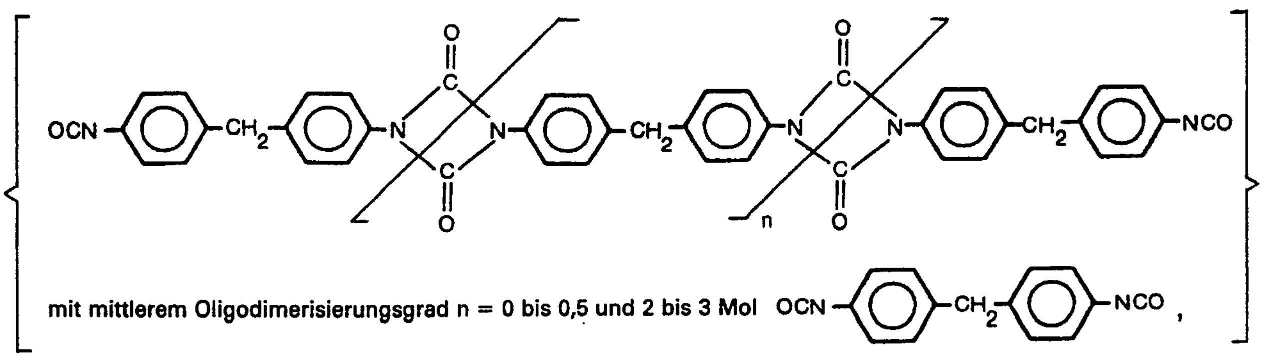

mit mittlerem Oligodimerisierungsgrad n = 0 bis 0,5 und 2 bis 3 Mol ,

aus 1 Mol eines niedermolekularen 4,4'-Diphenylmethan-uretdionisocyanats (Oligodimerisierungsgrad n = 0 bis 0,5) und 2 bis 3 Mol 4,4'-Diisocyanatodiphenylmethan, durch Dimerisierung von 4,4'-Diisocyanatodiphenylmethan in NCO-inerten Lösungsmitteln in Gegenwart von Dimerisierungskatalysatoren und gegebenenfalls Abstoppern, dadurch gekennzeichnet, daß man die Dimerisierung in einer Emulsion von fein verteiltem, flüssigem Diphenylmethan-4,4'-diisocyanat in einem unpolaren Lösungsmittel, das ein möglichst geringes Lösungsvermögen für die Startkomponente Diphenylmethan-4,4'-diisocyanat aufweist, als Emulgiermittel durchführt, wobei die Löslichkeit von Diphenylmethandiisocyanat bei Raumtemperatur in diesem Lösungsmittel höchstens 25 Gew.-% beträgt und daß die Dimerisierungsreaktion bei 40 bis 60°C gestartet wird und der zunächst leicht exotherm reagierende Ansatz während der Dimerisierung bei einer Temperatur von 0 bis 40°C gehalten wird.

2. Verfahren gemäß Anspruch 1, dadurch gekennzeichnet, daß in aliphatischen und/oder cycloaliphatischen Kohlenwasserstoffen, Kohlenwasserstoff-Destillaten mit überwiegendem Gehalt an aliphatischen und/oder cycloaliphatischen Kohlenwasserstoffen oder Ethern als Emulgiermittel dimerisiert wird.

3. Verfahren gemäß Anspruch 2, dadurch gekennzeichnet, daß die aliphatischen und/oder cycloaliphatischen Kohlenwasserstoffe oder Kohlenwasserstoff-Destillate mit überwiegendem Gehalt an aliphatischen und/oder cycloaliphatischen Kohlenwasserstoffen Dielektrozitätskonstanten von unter 2,10 aufweisen.

4. Verfahren gemäß Anspruch 1, dadurch gekennzeichnet, daß die Löslichkeit von Diphenylmethandiisocyanat bei Raumtemperatur in diesem Lösungsmittel 2 bis 15 Gew.-%, vorzugsweise 1 bis 12 Gew.-% beträgt, die Dimerisierungsreaktion 5—100 Minuten lang bei 40 bis 50°C gestartet und der zunächst leicht exotherm reagierende Ansatz während der Dimerisierung bei einer Temperatur von 15 bis 30°C gehalten oder bevorzugt ohne externe Heizung auf Raumtemperatur abgekühlt wird.

5. Additionsverbindung, erhältlich nach Verfahren der Ansprüche 1 bis 4, aus 1 Mol eines niedermolekularen 4,4'-Diphenylmethan-Uretdiondiisocyanats (I; n = 0 bis 0,5) und etwa 2 bis 3 Mol 4,4'-Diisocyanatodiphenylmethan (II), wobei der Gehalt dieser Additionsverbindung an freiem NCO im Bereich von 22,4 bis 26,9 Gew.-% liegt.

6. Additionsverbindung gemäß Anspruch 5 mit einem Gehalt an freiem NCO im Bereich von 24,4 bis 26 Gew.-%.

7. Verwendung der Additionsverbindung nach den Ansprüchen 5 und 6 als Polyisocyanatkomponente zum Aufbau von Polyurethanen, insbesondere lagerstabilen Einkomponentenpolyurethanreaktivmischungen.

**Revendications**

1. Procédé de production d'un composé d'addition d'un type nouveau de formule théorique (III)

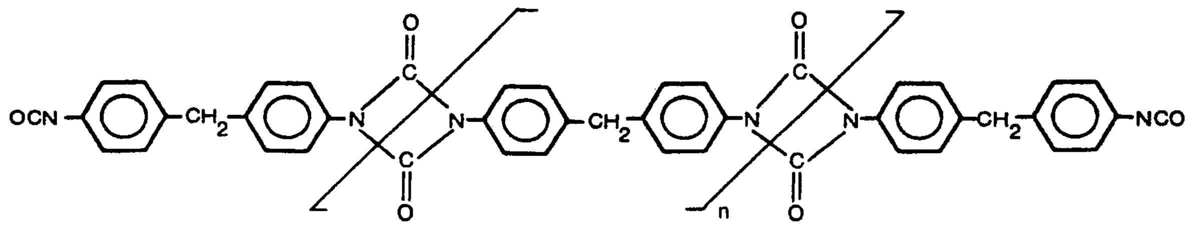

avec un degré moyen d'oligodimérisation n ayant une valeur de 0 à 0,5 et 2 à 3 moles de

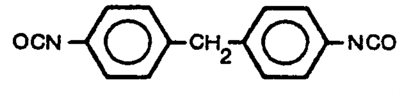

à partir d'une mole d'un 4,4'-diphénylméthane-uretdione-isocyanate de bas poids moléculaire (degré d'oligodimérisation n = 0 à 0,5) et 2 à 3 moles de 4,4'-diisocyanatodiphénylméthane, par dimérisation de 4,4'-diisocyanatodiphénylméthane dans des solvants inertes vis-à-vis de NCO en présence de catalyseurs de dimérisation et, le cas échéant, d'inhibiteurs, caractérisé en ce qu'on conduit la dimérisation dans une émulsion de 4,4'-diisocyanatodiphénylméthane liquide finement divisée dans un émulsionnant qui est un solvant non polaire présentant un pouvoir de dissolution aussi faible que possible envers le 4,4'-diisocyanatodiphénylméthane utilisé comme composant de départ, la solubilité du diisocyanatodiphénylméthane à la température ambiante dans ce solvant étant au maxium égale à 25% en poids, et en ce que la réaction de dimérisation est amorcée à 40—60°C et la charge qui a tout d'abord une réaction légèrement exothermique est maintenue pendant la dimérisation à une température de 0 à 40°C.

2. Procédé suivant la revendication 1, caractérisé en ce qu'on conduit la dimérisation dans des émulsionnants consistant en hydrocarbures aliphatiques et/ou cycloaliphatiques, en des distillats hydrocarbonés contenant principalement des hydrocarbures aliphatiques et/ou cycloaliphatiques, ou en éthers.

3. Procédé suivant la revendication 2, caractérisé en ce que les hydrocarbures aliphatiques et/ou cycloaliphatiques ou les distillats hydrocarbonés contenant principalement des hydrocarbures aliphatiques et/ou cycloaliphatiques présentent des constantes diélectriques inférieures à 2,10.

4. Procédé suivant la revendication 1, caractérisé en ce que la solubilité du diisocyanatodiphénylméthane à la température ambiante dans ce solvant est de 2 à 15% en poids, de préférence de 1 à 12% en poids, la réaction de dimérisation est amorcée pendant 5 à 100 minutes à 40—50°C et la charge qui a tout d'abord une réaction légèrement exothermique est maintenue pendant la dimérisation à une température de 15 à 30°C ou est refroidie à la température ambiante, de préférence sans chauffage externe.

5. Composé d'addition pouvant être obtenu par le procédé des revendications 1 à 4, à partir d'une mole d'un 4,4'-diphénylméthane-uretdione-diisocyanate de bas poids moléculaire (I; n = 0 à 0,5) et d'environ 2 à 3 moles de 4,4'-diisocyanatodiphénylméthane (II), la teneur de ce composé d'addition en NCO libre se situant dans l'intervalle de 22,4 à 26,9% en poids.

6. Composé d'addition suivant la revendication 5, ayant une teneur en NCO libre dans l'intervalle de 24,4 à 26% en poids.

7. Utilisation du composé d'addition suivant les revendications 5 et 6 comme composant polyisocyanate pour l'édification de polyuréthannes, notamment de mélanges réactifs pour polyuréthannes à un composant, stables à l'entreposage.

**Claims**

1. A process for the production of a new addition compound having the idealized formula (III)

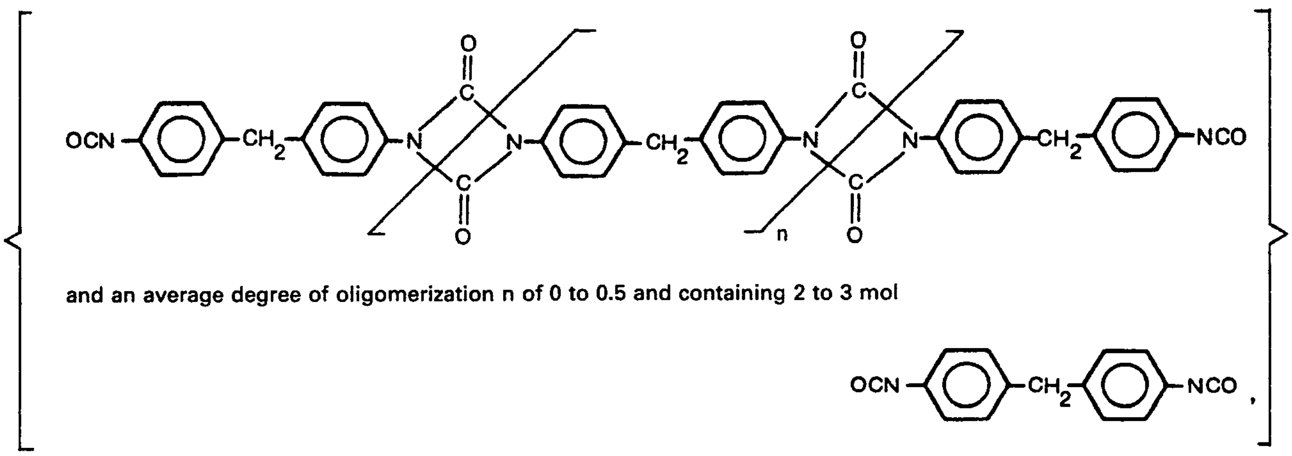

and an average degree of oligomerization n of 0 to 0.5 and containing 2 to 3 mol

of 1 mol of a low molecular weight 4,4'-diphenylmethane uretdione isocyanate (degree of oligomerization n = 0 to 0.5) and 2 to 3 mol 4,4'-diisocyanatodiphenyl methane by dimerization of 4,4'-diisocyanatodiphenyl methane in NCO-inert solvents in the presence of dimerization catalysts and optionally terminators, characterized in that the dimerization is carried out in an emulsion of finely divided, liquid diphenylmethane-4,4'-diisocyanate in an apolar solvent, which has minimal dissolving power for the starting component diphenylmethane-4,4'-diisocyanate, as emulsifier, the solubility of diphenylmethane diisocyanate in this solvent at room temperature being at most 25% by weight, and in that the dimerization reaction is initiated at 40 to 60°C and the initially slightly exothermically reacting mixture is kept at a temperature of 0 to 40°C during the dimerization.

2. A process as claimed in claim 1, characterized in that the dimerization is carried out in aliphatic and/or cycloaliphatic hydrocarbons, hydrocarbon distillates predominantly containing aliphatic and/or cycloaliphatic hydrocarbons or ethers as emulsifier.

3. A process as claimed in claim 2, characterized in that the aliphatic and/or cycloaliphatic hydrocarbons or hydrocarbon distillates predominantly containing aliphatic and/or cycloaliphatic hydrocarbons have dielectric constants below 2.10.

4. A process as claimed in claim 1, characterized in that the solubility of diphenylmethane diisocyanate in this solvent at room temperature amounts to between 2 and 15% by weight and preferably to between 1 and 12% by weight, the dimerization reaction is initiated for 5 to 100 minutes at 40 to 50°C and the initially slightly exothermically reacting mixture is kept at a temperature of 15 to 30°C during the dimerization or is cooled to room temperature, preferably without external heating.

5. An addition compound obtainable by the process claimed in claims 1 to 4 of 1 mol of a low molecular weight 4,4'-diphenylmethane uretdione diisocyanate (I; n = 0 to 0.5) and about 2 to 3 mol 4,4'-diisocyanatodiphenyl methane (II), the free NCO content of this addition compound being from 22.4 to 26.9% by weight.

6. The addition compound claimed in claim 6 with a free NCO content of from 24.4 to 26% by weight.

7. The use of the addition compound claimed in claims 5 and 6 as polyisocyanate component in the synthesis of polyurethanes, particularly storable one-component reactive polyurethane mixtures.